# EUROPEAN PATENT SPECIFICATION

(11) **EP 2 574 281 B1**
(45) Date of publication and mention of the grant of the patent: **24.12.2014**
(21) Application number: 12186450.8
(22) Date of filing: 27.09.2012
(51) Int. Cl.: A61B 6/10, A61B 6/14, F16M 11/08, A61B 6/00

(54) **Radiography apparatus**
Röntgengerät
Appareil de radiographie

(30) Priority: 27.09.2011 IT BO20110550
(43) Date of publication of application: 03.04.2013
(73) Proprietor: CEFLA SOCIETA' COOPERATIVA, 40026 Imola (BO) (IT)
(72) Inventor: Becca, Antonio, 40026 Imola (IT); Cavina, Massimo, 40026 Imola (IT)
(74) Representative: Bergadano, Mirko

(56) References cited:
- WO-A1-86/06950
- JP-A- 2010 259 780
- US-A- 5 784 435
- US-A- 6 088 424

## Description

The present invention concerns a radiography apparatus.

The present invention can be applied to particular advantage in the sector of radiography equipment for the acquisition of dental radiographic images, in particular cone beam computed tomography images (commonly known as CBCT), to which the following discussion will make explicit reference without loss of generality.

In the sector of dental radiography, a radiography apparatus is known of the type comprising a vertical upright extending upwards from the ground in a first direction; a vertical slide, which is mounted to perform vertical movements along the upright in the first direction, has a substantially L-shaped form and is provided with an upper arm protruding horizontally from the upright in a second direction; a first horizontal slide which moves along the upper arm in the second direction; and a second horizontal slide coupling in a sliding manner with the first horizontal slide to perform rectilinear movements in a third direction orthogonal to said first and second direction.

The second horizontal slide supports a radiographic unit comprising an elongated bracket, at the opposite free ends of which an X ray emitter and a receiver of the X rays generated by the emitter are fixed.

The bracket is coupled in a revolving manner, at an intermediate point thereof, to the second slide to rotate, with respect to the second slide, about an axis of rotation parallel to the first direction.

The known radiography apparatus of the type described above is used to acquire a series of two-dimensional radiographic images from different projection angles.

In order to correctly process the radiographic images in a post-acquisition phase and reconstruct a three-dimensional volume, a relatively broad field of view (FOV) of the patient's dental arches is desirable.

To avoid using relatively large and therefore excessively costly X ray receivers, the X ray cone beam is not centred with respect to the geometric centre of the area to be acquired, but is appropriately decentred so as to obtain a greater field of view.

Consequently, the acquisition involves a minimum rotation of 360° of the radiographic unit about the cited axis of rotation. In reality, to take account of the initial acceleration and final deceleration, the radiographic unit must rotate according to an angle greater than 360°, preferably between 370° and 380°, about the cited axis of rotation.

A rotation of the radiographic unit greater than 360° entails the presence of a stopper means suitable for preventing a rotation greater than the maximum and, therefore, mechanical damage to the radiography apparatus, in particular to the wiring which would be subject to excessive twisting and, at worst, could be pulled out.

The known radiography apparatus of the type described has some drawbacks mainly arising from the fact that the stopper means normally used in said radiography apparatus is an electronic detection system suitable for detecting the instantaneous angular position of the radiographic unit about the cited axis of rotation. Consequently, the stopper means is active only when it is electrically powered, i.e. when the radiography apparatus is switched on, whereas when the radiography apparatus is switched off, the stopper means is inactive and, therefore, unable to prevent pulling out of the wiring due to any manual movement of the radiographic unit.

The object of the present invention is to provide a radiography apparatus which is free from the drawbacks described above and is simple and inexpensive to produce.

According to the present invention there is provided a radiography apparatus as claimed in the attached claims.

Document US6088424 discloses an apparatus according to the preamble of claim 1.

The present invention will now be described with reference to the accompanying drawings, which illustrate a non-limiting implementation example, in which:
figure 1 is a schematic perspective view of a preferred embodiment of the radiography apparatus of the present invention;
figure 2 is a schematic perspective view of a detail of the radiographic apparatus of figure 1;
figure 3 is a schematic plan view, with parts removed for the sake of clarity, of a detail of figure 2;
figure 4 is a schematic lateral view, with parts removed for the sake of clarity, of the detail of figure 3;
figures 5 and 6 are two schematic plan views, with parts enlarged and parts removed for the sake of clarity, of the detail of figures 3 and 4 illustrated in two different operating positions; and
figure 7 is a schematic plan view of a detail of figures 3 to 6.

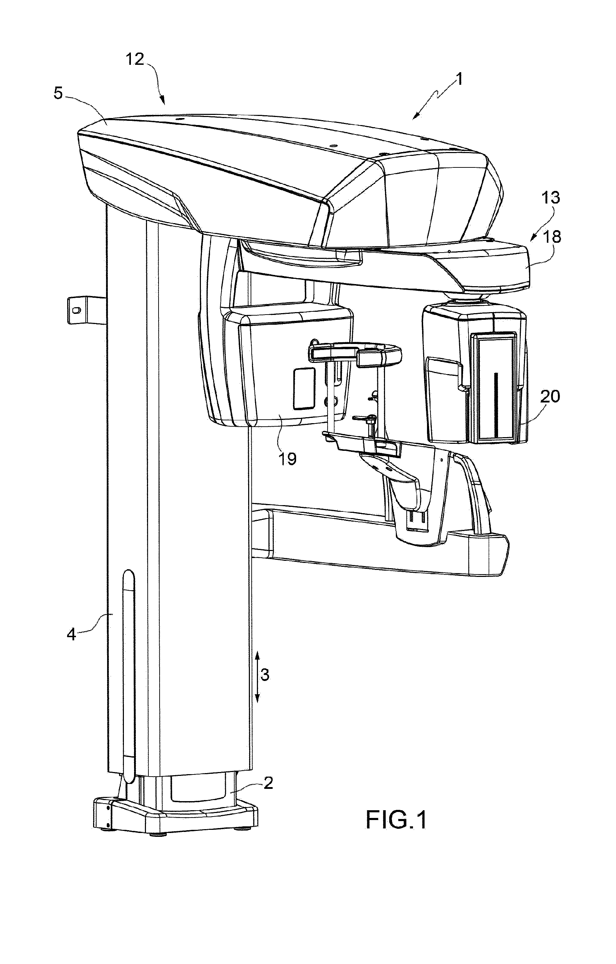

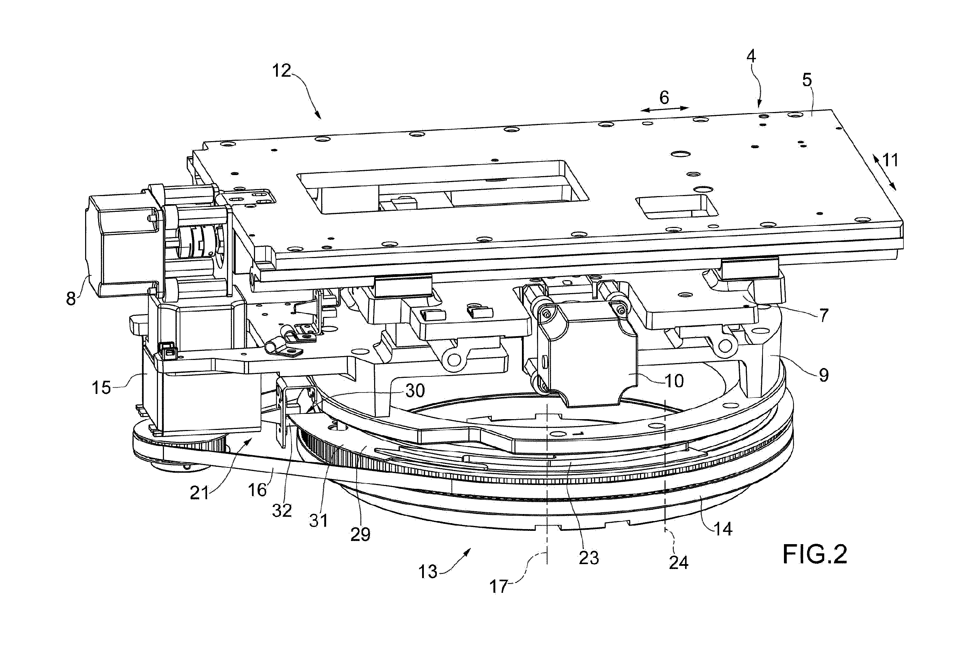

With reference to figures 1 and 2, the reference number 1 indicates, overall, a radiography apparatus, in this case a radiographic apparatus for the acquisition of CBCT images.

The radiography apparatus 1 comprises a fixed vertical upright 2, which extends upwards in a direction 3, and is engaged in a sliding manner by a vertical slide 4, which presents an L-shaped form with concavity facing down, and is movable along the upright 2 in the direction 3.

The slide 4 presents a horizontal cross member 5, which protrudes from the upright 2 in a horizontal direction 6 transverse to the direction 3, and supports a first horizontal slide 7 coupled in a sliding manner with the slide 4 to perform, with respect to the slide 4 and under the action of a drive device 8 of known type, rectilinear movements in the direction 6.

The slide 7 supports a second horizontal slide 9 coupled in a sliding manner with the slide 7 to perform, with respect to the slide 7 and under the action of a drive device 10 of known type, rectilinear movements in a horizontal direction 11 orthogonal to the directions 3 and 6.

The upright 2 and the slides 4, 7 and 9 define a frame 12 supporting a rotating radiographic unit 13 comprising a disc 14 coupled in a revolving manner with the slide 9 to rotate, with respect to the slide 9 and under the action of an electric motor 15 connected to the disc 14 by the interposition of a belt drive 16, around a rotation axis 17 parallel to the direction 3.

The disc 14 supports a bracket 18 of elongated shape, which is coupled centrally and angularly fixed to the disc 14, and bears, connected to its free ends, an X ray emitter 19 and a receiver 20 of the X rays emitted by the emitter 19.

The emitter 19 and the receiver 20 protrude downwards from the bracket 18, they face each other and are arranged on opposite sides of the axis 17.

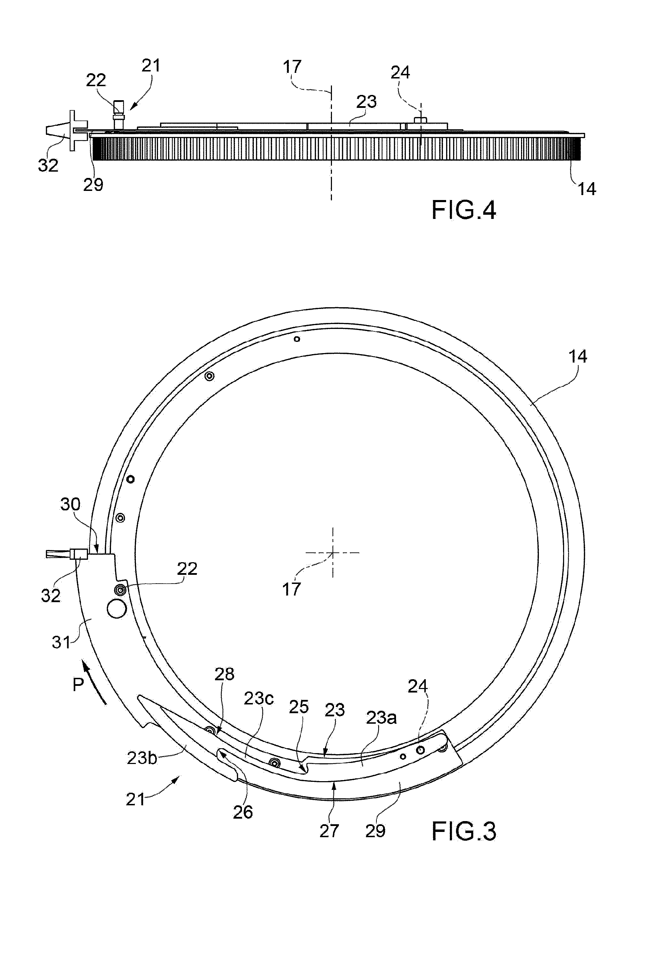

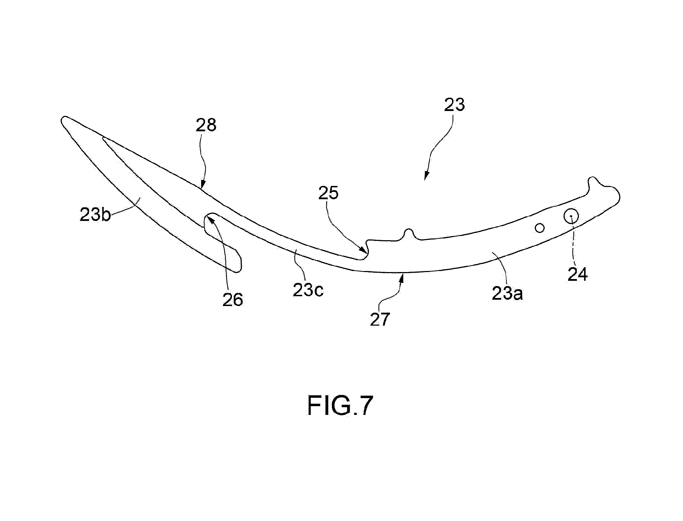

According to figures 3, 4 and 7, the movement of the unit 13 about the axis 17 is limited by a mechanical stopper means 21 comprising a stopper pin 22 protruding downwards from the slide 9 in the direction 3, and a stopper arm 23 mounted on an upper face of the disc 14.

The arm 23 has an elongated shape, extends around the axis 17, and is shaped so as to define two widened end portions 23a, 23b and a narrow central portion 23c arranged between the portions 23a and 23b.

The arm 23 is hinged to the disc 14 at the portion 23a to oscillate, with respect to the disc 14 and under the action of the pin 22, around a fulcrum axis 24 parallel to the axis 17 and the direction 3, and is moved, and normally maintained, in a rest position by a spring (not illustrated) positioned between the disc 14 and the arm 23.

The portions 23a, 23b protrude radially towards the inside and outside respectively of the portion 23c thus defining, together with the portion 23c, two seats 25, 26, which are obtained at the ends and on opposite sides of the portion 23c, they are offset from each other both radially and tangentially, and have respective concavities facing each other.

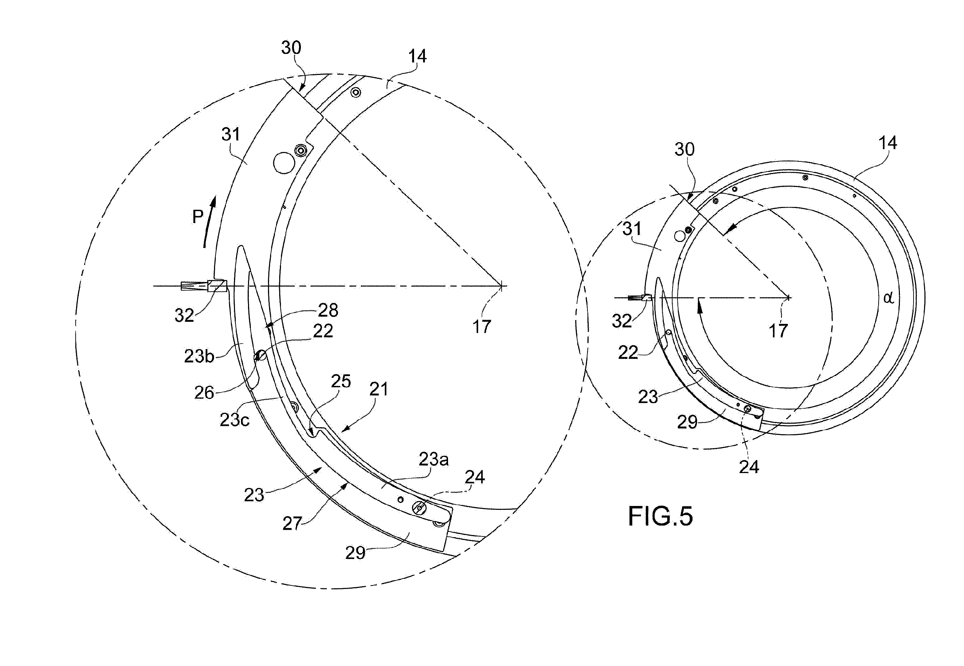

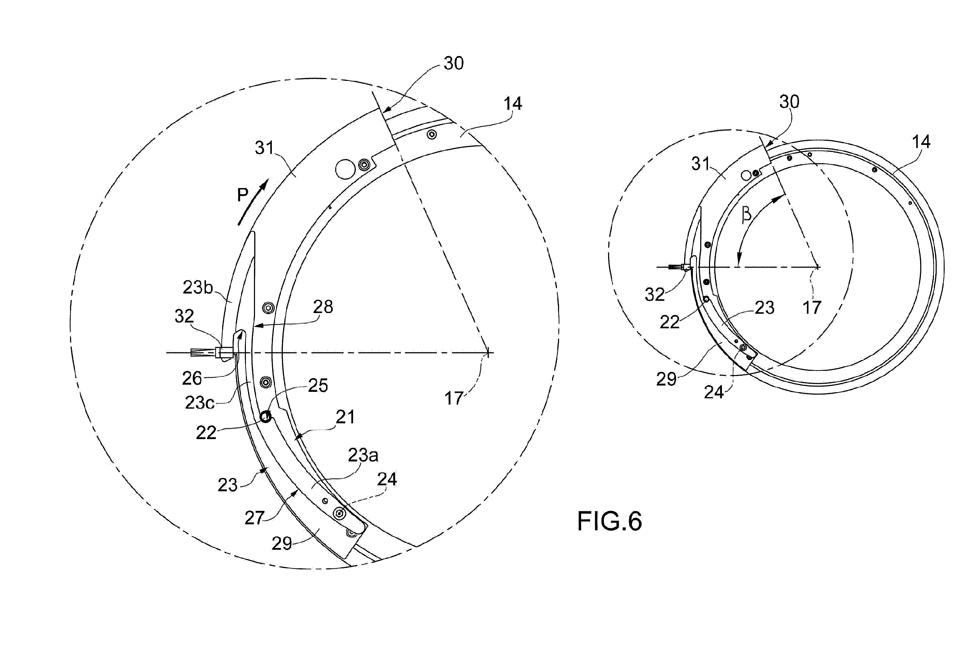

The portion 23c therefore connects with the portions 23a, 23b along respective surfaces 27, 28, of which the surface 27 delimits the seat 25 and the surface 28 delimits the seat 26. In use, rotation of the unit 13 in a clockwise direction (figures 3 and 6) is limited by engagement of the pin 22 in the seat 25, while rotation of the unit 13 in an anticlockwise direction (figures 3 and 5) is limited by engagement of the pin 22 in the seat 26.

With regard to the above it should be noted that:
the stopper means 21 limits the rotation of the unit 13 about the axis 17 both when the radiography apparatus 1 is switched on and when the radiography apparatus 1 is switched off; and
the position and orientation of the seats 25, 26 and the oscillation of the arm 23 around the axis 24 allow the unit 13 to rotate about the axis 17 according to an angle greater than 360°, in this case according to an angle substantially equal to 380°.

The unit 13 is furthermore provided with a reference blade 29, which is fixed to the upper face of the disc 14 below the arm 23, and extends around the axis 17.

The blade 29 protrudes tangentially from the arm 23, has a flat end face 30 substantially perpendicular to a direction of forward movement of the unit 13 around the axis 17, and comprises a portion 31 protruding radially from the perimeter edge of the disc 14.

The radiography apparatus 1 furthermore comprises a detection device defined, in this case, by a photocell 32, which is fixed to the slide 9 in a position facing the disc 14, and is arranged along a path P of forward movement of the portion 31.

In this regard it should be noted that:
rotation of the unit 13 in a clockwise direction allows the pin 22 to engage the surface 28 and to move the arm 23 to a first operating position, in which the portion 23b protrudes radially towards the outside of the disc 14 thus defining an extension of the blade 29 and crossing the photocell 32; and
the rotation of the unit 13 in an anticlockwise direction allows the pin 22 to engage the surface 27 and to move the arm 23 to a second operating position, in which the arm 23 does not protrude radially towards the outside of the disc 14 and does not cross the photocell 32.

The rotation of the unit 13 about the axis 17 is commanded from an initial reference position (commonly called "machine 0 position") defined at the moment when the photocell 32 is activated or de-activated by the passage of the face 30 (figure 3).

When the radiography apparatus 1 is switched on, the unit 13 is generally set to an indefinite angular position around the axis 17 and must be firstly moved to its initial reference position. Consequently, if the photocell 32 is intercepted by the blade 29 or by the portion 23b, the unit 13 is rotated in an anticlockwise direction until the face 30 uncovers the photocell 32, whereas if the photocell 32 is not intercepted either by the blade 29 or by the portion 23b, the unit 13 is rotated in a clockwise direction until the face 30 covers the photocell 32.

Figure 5 shows the anticlockwise stopper position, in which the unit 13 has moved about the axis 17 according to an angle α starting from the initial reference position and the pin 22 has stopped against the seat 26. In this position, the photocell 32 is uncovered since the arm 23 is set to its second operating position and the portion 31 is sized so as to extend upstream of the photocell 32.

Figure 6 shows the clockwise stopper position, in which the unit 13 has moved around the axis 17 according to an angle β starting from the initial reference position and the pin 22 has stopped against the seat 25. In this position, the photocell 32 is covered since the arm 23 is set to its first operating position.

In other words:
the unit 13 can move around the axis 17 according to an overall angle α+β greater than 360°, in this case according to an overall angle substantially equal to 380°;
the combination of the arm 23, the blade 29 and the photocell 32 allows recognition of the angular position of the unit 13 when the radiography apparatus 1 is switched on and re-setting of the unit 13 always to the same identical initial reference position; and
the arm 23, together with the pin 22, acts as a stopper means and, together with the blade 29 and the photocell 32, as a device for recognition of the initial angular position of the unit 13 around the axis 17.

According to a variation not illustrated, the blade 29 is eliminated and the arm 23 is shaped in order to perform also the function of the blade 29.

## Claims

1. Radiography apparatus comprising a first supporting frame (12); a radiographic unit (13) mounted on the first supporting frame (12) and comprising, in turn, a second supporting frame (14, 18) mounted to rotate, with respect to the first supporting frame (12), about a rotation axis (17), an X ray emitter (19) mounted on the second supporting frame (14, 18), and an X ray receiver (20) mounted on the second supporting frame (14, 18) to receive the X rays generated by the emitter (19); and a stopper means (21) to stop rotation of the radiographic unit (13) about said rotation axis (17);
wherein
the stopper means (21) comprises a stopper pin (22) mounted on one of said first and second supporting frames (12, 14, 18) and **characterised by** one single stopper arm (23) mounted on the other of said first and second supporting frames (12, 14, 18); the stopper arm (23) having two seats (25, 26) suitable for being selectively engaged by the stopper pin (22) following rotation of the second supporting frame (14, 18) in two opposite directions of forward movement.

2. Radiography apparatus as claimed in claim 1, wherein the two seats (25, 26) are radially and tangentially offset from each other with respect to the rotation axis (17) and have respective concavities facing each other so as to allow the second supporting frame(14, 18) to move around the rotation axis (17) according to an angle greater than 360°.

3. Radiography apparatus as claimed in claim 1 or 2, wherein the two seats (25, 26) are radially offset from each other with respect to said rotation axis (17); the stopper arm (23) being movable, with respect to the second supporting frame (14, 18), between a first operating position, in which the stopper pin (22) engages a first said seat (25) following rotation of the second supporting frame (14, 18) in a first said direction of forward movement, and a second operating position wherein the stopper pin (22) engages a second said seat (26) following rotation of the second supporting frame (14, 18) in a second said direction of forward movement.

4. Radiography apparatus as claimed in claim 3, wherein the stopper arm (23) is hinged to the second supporting frame (14, 18) to rotate about a fulcrum axis (24) substantially parallel to said rotation axis (17) between said first and second operating position under the thrust of said stopper pin (22).

5. Radiography apparatus as claimed in claim 3 or 4 and furthermore comprising at least one spring positioned between the second supporting frame (14, 18) and the stopper arm (23) to move, and normally maintain, the stopper arm (23) in an intermediate rest position between said first and second operating position.

6. Radiography apparatus as claimed in any one of the preceding claims, wherein the stopper arm (23) is shaped so as to extend around said rotation axis (17).

7. Radiography apparatus as claimed in any one of the preceding claims and furthermore comprising a reference member (23, 29) mounted on the second supporting frame (14, 18) to move along a path of forward movement (P) extending around the rotation axis (17), and a detection device (32) positioned along the path of forward movement (P) to detect the presence of the reference member (23, 29).

8. Radiography apparatus as claimed in claim 7, wherein the reference member (23, 29) extends around the rotation axis (17) according to a given angle, and has at least one reference element (30); the detection device (32) being configured to command stopping of the radiographic unit (13) around the rotation axis (17) in an angular position corresponding to the passage of the reference element (30) through the detection device (32).

9. Radiography apparatus as claimed in claim 8, wherein the reference member (23, 29) is limited tangentially by a substantially flat free face defining said reference element (30).

10. Radiography apparatus as claimed in any one of the claims from 7 to 9, wherein the stopper arm (23) defines said reference member (23, 29).

11. Radiography apparatus as claimed in any one of the claims from 7 to 9, wherein the reference member (23, 29) comprises a first portion defined by a blade (29), which extends around the rotation axis (17), is fixed to the second supporting frame (14, 18) and protrudes radially towards the outside of the second supporting frame (14, 18) to cross the detection device (32).

12. Radiography apparatus as claimed in claim 11, wherein the reference member (23, 29) comprises a second portion defined by the stopper arm (23).

13. Radiography apparatus as claimed in claim 12 when dependent on any one of the claims from 3 to 5, wherein the stopper arm (23) is shaped so as to define an extension of the blade (29) and cross the detection device (32) when arranged in said first operating position.

14. Radiography apparatus as claimed in claim 13, wherein the stopper arm (23) is shaped so as not to cross the detection device (32) when arranged in said second operating position.

15. Radiography apparatus as claimed in claim 14, wherein the blade (29) extends around the rotation axis (17) according to an angle such as to extend to the outside of the detection device (32) when the stopper arm (23) is arranged in said second operating position.

## Patentansprüche

1. Röntgengerät mit einem ersten Stützrahmen (12); einer an dem ersten Stützrahmen (12) befestigten Röntgeneinheit (13), die ihrerseits einen zweiten Stützrahmen (14, 18), welcher in Bezug auf den ersten Stützrahmen (12) um eine Drehachse (17) drehbar angebracht ist, einen an dem zweiten Stützrahmen (14, 18) angebrachten Röntgenstrahlemitter (19), und einen an dem zweiten Stützrahmen (14, 18) angebrachten Röntgenstrahlempfänger (20) zum Empfangen der von dem Emitter (19) erzeugten Röntgenstrahlen aufweist; und einer Anschlageinrichtung (21) zum Anhalten des Drehens der Röntgeneinheit (13) um die Drehachse (17);
wobei die Anschlageinrichtung (21) einen an dem ersten oder dem zweiten Stützrahmen (12, 14, 18) angebrachten Anschlagstift (22) aufweist, und
**gekennzeichnet durch**
einen einzelnen Anschlagarm (23), welcher an dem anderen Stützrahmen, dem ersten oder dem zweiten Stützrahmen (12, 14, 18), angebracht ist; wobei der Anschlagarm (23) zwei Aufnahmen (25, 26) aufweist, in welche der Anschlagstift (22) nach dem Drehen des zweiten Stützrahmens (14, 18) in zwei entgegengesetzte Vorwärtsbewegungsrichtungen wahlweise eingreifen kann.

2. Röntgengerät nach Anspruch 1, bei welchem die beiden Aufnahmen (25, 26) in Bezug auf die Drehachse (17) radial und tangential zueinander versetzt sind und jeweilige Vertiefungen aufweisen, die einander zugewandt sind, so dass ein Bewegen des zweiten Stützrahmens (14, 18) um die Drehachse (17) über einen Winkel von mehr als 360° möglich ist.

3. Röntgengerät nach Anspruch 1 oder 2, bei welchem die beiden Aufnahmen (25, 26) in Bezug auf die Drehachse (17) radial zueinander versetzt sind; wobei der Anschlagarm (23) in Bezug auf den zweiten Stützrahmen (14, 18) zwischen einer ersten Betriebsposition, in welcher der Anschlagstift (22) nach dem Drehen des zweiten Stützrahmens (14, 18) in eine erste Vorwärtsbewegungsrichtung in die erste Aufnahme (25) eingreift, und einer zweiten Betriebsposition bewegbar ist, in welcher der Anschlagstift (22) nach dem Drehen des zweiten Stützrahmens (14, 18) in eine zweite Vorwärtsbewegungsrichtung in die zweite Aufnahme (26) eingreift.

4. Röntgengerät nach Anspruch 3, bei welchem der Anschlagarm (23) gelenkig mit dem zweiten Stützrahmen (14, 18) verbunden ist, um unter Schubwirkung des Anschlagstifts (22) um eine Gelenkachse (24), die im Wesentlichen parallel zur Drehachse (17) verläuft, zwischen der ersten und der zweiten Betriebsposition zu drehen.

5. Röntgengerät nach Anspruch 3 oder 4 und ferner mit mindestens einer zwischen dem zweiten Stützrahmen (14, 18) und dem Anschlagarm (23) angeordneten Feder zum Bewegen, und normalerweise Halten, des Anschlagarms (23) in einer Ruhe-Zwischenposition zwischen der ersten und der zweiten Betriebsposition.

6. Röntgengerät nach einem der vorhergehenden Ansprüche, bei welchem der Anschlagarm (23) derart geformt ist, dass er sich um die Drehachse (17) erstreckt.

7. Röntgengerät nach einem der vorhergehenden Ansprüche und ferner mit einem Referenzteil (23, 29), das an dem zweiten Stützrahmen (14, 18) angebracht ist, um sich entlang eines um die Drehachse (17) verlaufenden Vorwärtsbewegungswegs (P) zu erstrecken, und einer Detektionsvorrichtung (32), die entlang dem Vorwärtsbewegungsweg (P) angeordnet ist, um das Vorhandensein des Referenzteils (23, 29) zu erkennen.

8. Röntgengerät nach Anspruch 7, bei welchem sich das Referenzteil (23, 29) über einen definierten Winkel um die Drehachse (17) erstreckt, und mindestens ein Referenzelement (30) aufweist; wobei die Detektionsvorrichtung (32) ausgebildet ist, um das Anhalten der Röntgeneinheit (13) um die Drehachse (17) herum in einer Winkelposition zu veranlassen, welche dem Durchgang des Referenzelements (30) durch die Detektionsvorrichtung (32) entspricht.

9. Röntgengerät nach Anspruch 8, bei welchem das Referenzteil (23, 29) tangential durch eine im Wesentlichen flache freie Fläche begrenzt ist, welche das Referenzelement (30) bildet.

10. Röntgengerät nach einem der Ansprüche 7 bis 9, bei welchem der Anschlagarm (23) das Referenzteil (23, 29) bildet.

11. Röntgengerät nach einem der Ansprüche 7 bis 9, bei welchem das Referenzelement (23, 29) einen ersten, durch eine Klinge (29) gebildeten Bereich aufweist, der sich um die Drehachse (17) erstreckt, an dem zweiten Stützrahmen (14, 18) angebracht ist und radial in Richtung der Außenseite des zweiten Stützrahmens (14, 18) hervorsteht, so dass er die Detektionsvorrichtung (32) durchquert.

12. Röntgengerät nach Anspruch 11, bei welchem das Referenzteil (23, 29) einen durch den Anschlagarm (23) gebildeten zweiten Bereich aufweist.

13. Röntgengerät nach Anspruch 12, sofern auf einen der Ansprüche 3 bis 5 bezogen, bei welchem der Anschlagarm (23) derart ausgebildet ist, dass er eine Verlängerung der Klinge (29) bildet und die Detektionsvorrichtung (32) durchquert, wenn er in der ersten Betriebsposition angeordnet ist.

14. Röntgengerät nach Anspruch 13, bei welchem der Anschlagarm (23) derart ausgebildet ist, dass er die Detektionsvorrichtung (32) nicht durchquert, wenn er sich in der zweiten Betriebsposition befindet.

15. Röntgengerät nach Anspruch 14, bei welchem die Klinge (29) sich um die Drehachse (17) über einen Winkel derart erstreckt, dass sie sich zur Außenseite der Detektionsvorrichtung (32) erstreckt, wenn sich der Anschlagarm (23) in der zweiten Betriebsposition befindet.

## Revendications

1. Appareil de radiographie comprenant une première structure de support (12) ; une unité radiographique (13) montée sur la première structure de support (12) et comprenant, à son tour, une deuxième structure de support (14, 18) montée de manière à tourner, par rapport à la première structure de support (12), autour d'un axe de rotation (17), un émetteur de rayons X (19) monté sur la deuxième structure de support (14, 18), et un récepteur de rayons X (20) monté sur la deuxième structure de support (14, 18) afin de recevoir les rayons X générés par l'émetteur (19) ; un moyen d'arrêt (21) pour arrêter une rotation de l'unité radiographique (13) autour dudit axe de rotation (17) ; **caractérisé par le fait que** le moyen d'arrêt (21) comprend une broche d'arrêt (22) montée sur une première structure parmi lesdites première et deuxième structures de support (12, 14, 18) et où un seul bras d'arrêt(23) monté sur l'autre structure parmi lesdites première et deuxième structures de support (12, 14, 18) ; le bras d'arrêt (23) présentant deux sièges (25, 26) adaptés pour être engagés de manière sélective par la broche d'arrêt (22) après une rotation de la deuxième structure de support (14, 18) selon deux sens opposés de déplacement de progression.

2. Appareil de radiographie selon la revendication 1, dans lequel les deux sièges (25, 26) sont espacés l'un de l'autre de manière radiale et de manière tangentielle par rapport à l'axe de rotation (17) et présentent des concavités respectives se faisant face l'une l'autre de manière à permettre à la deuxième structure de support (14, 18) de se déplacer autour de l'axe de rotation (17) selon un angle supérieur à 360°.

3. Appareil de radiographie selon la revendication 1 ou 2, dans lequel les deux sièges (25, 26) sont espacés l'un de l'autre de manière radiale par rapport audit axe de rotation (17) ; le bras d'arrêt (23) étant mobile, par rapport à la deuxième structure de support (14, 18), entre une première position de mise en oeuvre, dans laquelle la broche d'arrêt (22) engage un premier dit siège (25) après une rotation de la deuxième structure de support (14, 18) selon un premier dit sens de déplacement de progression, et une deuxième position de mise en oeuvre dans laquelle la broche d'arrêt (22) engage un deuxième dit siège (26) après une rotation de la deuxième structure de support (14, 18) selon un deuxième dit sens de déplacement de progression.

4. Appareil de radiographie selon la revendication 3, dans lequel le bras d'arrêt (23) est articulé sur la deuxième structure de support (14, 18) pour tourner autour d'un axe d'articulation (24) sensiblement parallèle audit axe de rotation (17) entre lesdites première et deuxième positions de mise en oeuvre sous la poussée de ladite broche d'arrêt (22).

5. Appareil de radiographie selon la revendication 3 ou 4, et comprenant en outre au moins un ressort positionné entre la deuxième structure de support (14, 18) et le bras d'arrêt (23) pour déplacer, et en principe maintenir, le bras d'arrêt (23) dans une position de repos intermédiaire entre lesdites première et deuxième positions de mise en oeuvre.

6. Appareil de radiographie selon l'une quelconque des revendications précédentes, dans lequel le bras d'arrêt (23) est façonné de manière à s'étendre autour dudit axe de rotation (17).

7. Appareil de radiographie selon l'une quelconque des revendications précédentes, et comprenant en outre un élément de référence (23, 29) monté sur la deuxième structure de support (14, 18) de manière à se déplacer le long d'un trajet de déplacement de progression (P) s'étendant autour de l'axe de rotation (17), et un dispositif de détection (32) positionné le long du trajet de déplacement de progression (P) pour détecter la présence de l'élément de référence (23, 29).

8. Appareil de radiographie selon la revendication 7, dans lequel l'élément de référence (23, 29) s'étend autour de l'axe de rotation (17) selon un angle donné, et présente au moins un point de référence (30) ; le dispositif de détection (32) étant configuré pour commander l'arrêt de l'unité radiographique (13) autour de l'axe de rotation (17) dans une position angulaire correspondant au passage du point de référence (30) au travers du dispositif de détection (32).

9. Appareil de radiographie selon la revendication 8, dans lequel l'élément de référence (23, 29) est limité de manière tangentielle par une surface dégagée sensiblement plane définissant ledit point de référence (30).

10. Appareil de radiographie selon l'une quelconque des revendications 7 à 9, dans lequel le bras d'arrêt (23) définit ledit élément de référence (23, 29).

11. Appareil de radiographie selon l'une quelconque des revendications 7 à 9, dans lequel l'élément de référence (23, 29) comprend une première partie définie par une lame (29), laquelle s'étend autour de l'axe de rotation (17), est fixée à la deuxième structure de support (14, 18) et dépasse de manière radiale vers l'extérieur de la deuxième structure de support (14, 18) pour traverser le dispositif de détection (32).

12. Appareil de radiographie selon la revendication 11, dans lequel l'élément de référence (23, 29) comprend une deuxième partie définie par le bras d'arrêt (23).

13. Appareil de radiographie selon la revendication 12, lorsqu'elle dépend de l'une quelconque des revendications 3 à 5, dans lequel le bras d'arrêt (23) est façonné de manière à définir un prolongement de la lame (29) et à traverser le dispositif de détection (32) lorsqu'il est agencé dans ladite première position de mise en oeuvre.

14. Appareil de radiographie selon la revendication 13, dans lequel le bras d'arrêt (23) est façonné de manière à ne pas traverser le dispositif de détection (32) lorsqu'il est agencé dans ladite deuxième position de mise en oeuvre.

15. Appareil de radiographie selon la revendication 14, dans lequel la lame (29) s'étend autour de l'axe de rotation (17) selon un certain angle de telle sorte à s'étendre jusqu'à l'extérieur du dispositif de détection (32) lorsque le bras d'arrêt (23) est agencé dans ladite deuxième position de mise en oeuvre.
